# EUROPÄISCHE PATENTSCHRIFT

(11) **EP 0 761 641 B1**
(45) Veröffentlichungstag und Bekanntmachung des Hinweises auf die Patenterteilung: **21.03.2001**
(21) Anmeldenummer: 96113307.1
(22) Anmeldetag: 19.08.1996
(51) Int. Cl.: C07C 69/92, C07D 311/54, C07D 207/46, C07D 249/18, C08G 69/26

(54) **Dicarbonsäurederivate und ihre Verwendung zur Herstellung von Poly-o-hydroxyamiden oder Poly-o-mercaptoamiden**
Dicarboxylic acid derivatives and their use in the preparation of poly-o-hydroxyamides or poly-o-mercaptoamides
Dérivés d'acide dicarboxylique et leur application pour la préparation de poly-o-hydroxyamides ou poly-o-mercaptoamides

(30) Priorität: 31.08.1995 DE 19532138
(43) Veröffentlichungstag der Anmeldung: 12.03.1997
(73) Patentinhaber: Infineon Technologies AG, 81669 München (DE)
(72) Erfinder: Sezi, Recai, Dr., 91341 Röttenbach (DE); Ahne, Hellmut, Dr., 91341 Röttenbach (DE); Kühn, Eberhard, 91334 Hemhofen (DE)
(74) Vertreter: Zedlitz, Peter, Dipl.-Inf.

(56) Entgegenhaltungen:
- EP-A- 0 391 196
- CHEMICAL ABSTRACTS, vol. 92, no. 25, 23.Juni 1980 Columbus, Ohio, US; abstract no. 215344, KORSHAK, V. V. ET AL: "Synthesis of aromatic bibenzimidazoles by reduction heterocyclization" XP002020319 & IZV. AKAD. NAUK GRUZ. SSR, SER. KHIM. (1979), 5(3), 210-16,
- CHEMICAL ABSTRACTS, vol. 92, no. 21, 26.Mai 1980 Columbus, Ohio, US; abstract no. 181056, KORSHAK, V. V. ET AL: "Synthesis of aromatic bis(benzoxazoles) by reduction heterocyclization" XP002020320 & KHIM. GETEROTSIKL. SOEDIN. (1979), (12), 1620-3,
- CHEMICAL ABSTRACTS, vol. 84, no. 22, 31.Mai 1976 Columbus, Ohio, US; abstract no. 151032, WALTON, T. R. ET AL: "Phthalocyanine resins: A new class of thermally stable resins for adhesives, coatings, and plastics" XP002020321 & POLYM. SCI. TECHNOL. (1975), 9B(ADHES. SCI. TECHNOL.), 665-76,
- CHEMICAL ABSTRACTS, vol. 83, no. 5, 4.August 1975 Columbus, Ohio, US; abstract no. 43323, LISUNOVA, V. V. ET AL: "4,4'-Bis(aminobenzimidazole) bridged analogs of biphenyl" XP002020322 & SU 462 823 A (LENSOVET TECHNOLOGICAL INSTITUTE, LENINGRAD, USSR)
- CHEMICAL ABSTRACTS, vol. 82, no. 10, 10.März 1975 Columbus, Ohio, US; abstract no. 58354, ADROVA, N. A. ET AL: "Synthesis of poly(amide imides) and polyester amide imides from aromatic diamines containing amide bonds" XP002020323 & VYSOKOMOL. SOEDIN., SER. B (1974), 16(10), 788-90,

## Beschreibung

Die Erfindung betrifft neue Dicarbonsäurederivate, und zwar Diester, Dithioester und Diamide.

In der Mikroelektronik werden hochwärmebeständige Polymere benötigt, insbesondere als Schutz- und Isolierschichten bzw. als Dielektrika (siehe dazu beispielsweise: "SAMPE Journal", Vol. 25 (1989), No. 6, Seiten 18 bis 23, und "Proceedings of the 1992 International Conference on Multichip Modules", Seiten 394 bis 400). Einige der verwendeten Polymere, beispielsweise Homo- und Copolymere aromatischer Polyether sowie Vorstufen von Polyimiden (PI) und Polybenzoxazolen (PBO), weisen eine gute Löslichkeit in organischen Lösungsmitteln und gute Filmbildungseigenschaften auf und können mittels Schleudertechnik auf elektronische Bauteile aufgebracht werden (siehe dazu beispielsweise: "High Performance Polymers", Vol. 4 (1992), No. 2, Seiten 73 bis 80, und "Polymers for Advanced Technologies", Vol. 4 (1993), Seiten 217 bis 233).

Polymer-Vorstufen der genannten Art werden durch eine Temperaturbehandlung cyclisiert, d.h. in die entsprechenden Polymere (PI bzw. PBO) umgewandelt; dabei ergeben sich die endgültigen Eigenschaften. Durch die Cyclisierung verschwinden nämlich die hydrophilen Gruppierungen des Poly-o-hydroxyamids, d.h. die NH-, OH- und CO-Gruppen, welche die dielektrischen Eigenschaften und die Wasseraufnahme negativ beeinflussen würden. Dies ist beispielsweise ein wesentlicher Vorteil der Polybenzoxazole gegenüber den Polyimiden (mit zwei CO-Gruppen pro Imideinheit) und insbesondere gegenüber den Hydroxypolyimiden (mit zwei CO-Gruppen und einer OH-Gruppe pro Imideinheit). Außerdem ist die Cyclisierung nicht nur für die guten dielektrischen Eigenschaften und die geringe Wasseraufnahme des Endproduktes wichtig, sondern auch für dessen hohe Temperaturstabilität.

PI- und PBO-Vorstufen können, beispielsweise durch Zusatz geeigneter photoaktiver Komponenten, photosensitiv eingestellt und dann direkt strukturiert werden, d.h. ohne die Verwendung eines Hilfsresists. Dies ist deswegen bedeutsam, weil die direkte Strukturierung - im Vergleich mit der indirekten Strukturierung - erhebliche Kostenvorteile bietet.

Photosensitive PBO-Vorstufen bieten, im Gegensatz zu den meisten photosensitiven PI-Vorstufen, die Vorteile der positiven Strukturierbarkeit, wie geringere Defektdichte bei der Strukturierung der sogenannten "via holes", weil - im Vergleich mit negativ arbeitenden Systemen - nur ein Bruchteil der Fläche belichtet wird. Beim Einsatz alkalilöslicher PBO-Vorstufen ergibt sich außerdem die Möglichkeit einer wäßrig-alkalischen Entwicklung. Nach der Photostrukturierung erfolgt dann die Cyclisierung der Vorstufen durch Temperung.

Wäßrig-alkalisch entwickelbare PBO-Vorstufen sind bereits bekannt (siehe dazu: EP-PS 0 023 662, EP-OS 0 264 678 und EP-PS 0 291 779). Der dabei angewendete photolithographische Prozeß ist, bis auf die Cyclisierung, der gleiche wie bei der Strukturierung bekannter Positivresists auf der Basis von Novolaken und Chinonaziden, eines weltweit in zahlreichen Fertigungslinien eingesetzten Prozesses (siehe dazu beispielsweise: D.S. Soane und Z. Martynenko "Polymers in Microelectronics - Fundamentals and Applications", Elsevier, Amsterdam 1989, Seiten 77 bis 124).

Die Alkalilöslichkeit der PBO-Vorstufen ist eine wichtige Voraussetzung für deren Einsatz als Basispolymer für wäßrig-alkalisch entwickelbare photosensitive Dielektrika. Für mikroelektronische Anwendungen müssen die Vorstufen in metallionenfreien Entwicklern löslich sein, damit derartige Entwickler auch bei der Photostrukturierung verwendet werden können. Entwickler, welche Metallionen enthalten, können nämlich die elektrische Funktion der Bauteile negativ beeinflussen.

Die gängigste Methode zur Herstellung von alkalilöslichen PBO-Vorstufen, d.h. von Poly-o-hydroxyamiden, ist die Umsetzung eines Dicarbonsäurechlorids mit einem geeigneten Bis-o-aminophenol. Zum Abfangen des bei der Reaktion entstehenden Chlorwasserstoffs wird in der Regel eine lösliche Base, wie Pyridin, zugesetzt (siehe: EP-OS 0 264 678 und EP-PS 0 291 779). Nach dieser Methode sind nun zwar Vorstufen herstellbar, welche in metallionenfreien wäßrig-alkalischen Entwicklern löslich sind, von Nachteil ist jedoch, daß Chloridionen im Polymer verbleiben. Ein derartiges Polymer ist aber als Beschichtungsmaterial für mikroelektronische Bauteile nicht brauchbar, weil die Chloridionen Korrosion verursachen und die Funktion der Bauteile somit stark beeinträchtigen können. Es ist deshalb eine Reinigung des Polymers mittels Ionenaustauscher erforderlich. Diese Reinigung ist jedoch aufwendig und teuer, sie umfaßt nämlich zusätzliche Prozeßschritte, wie die Vorbereitung der Ionenaustauschersäule, das Lösen des Polymers, das Durchlaufen der Lösung durch die Säule und das Nachwaschen sowie die erneute Ausfällung und Trocknung.

Bei der Herstellung von Poly-o-hydroxyamiden muß auch die Forderung erfüllt werden, daß das Dicarbonsäurechlorid vorwiegend mit den Aminogruppen des Bis-o-aminophenols reagiert (unter Amidbildung), nicht aber mit dessen Hydroxylgruppen (unter Esterbildung), d.h. die Reaktionsselektivität der Amidbildung gegenüber der Esterbildung muß hoch sein. Kann die Esterbildung nicht ausgeschlossen bzw. stark unterdrückt werden, dann führt dies zu unzureichend alkalilöslichen Polymeren. Eine geringe Reaktionsselektivität kann ferner zu einer Gelbildung in der Polymerlösung führen, wobei das hergestellte Poly-o-hydroxyamid dann unfiltrierbar und somit unbrauchbar ist.

Es wurden auch bereits Verfahren zur chloridfreien Synthese von Poly-o-hydroxyamiden - und ebenso von Poly-o-mercaptoamiden - beschrieben. So ist es aus der EP-OS 0 158 726 bekannt, Dihydroxy- bzw. Dimercaptodiaminoverbindungen in Gegenwart eines Carbodiimids mit einer Dicarbonsäure umzusetzen. Bei dieser Reaktion bereiten jedoch Harnstoffreste, die aufgrund einer Umlagerungsreaktion am Harz verbleiben, oft Probleme. Sie beeinträchtigen nämlich die thermische Beständigkeit des Polybenzoxazols bzw. Polybenzothiazols sowie die Qualität der daraus hergestellten Schichten. Außerdem sind die nach diesem Verfahren hergestellten Polymere in metallionenfreien wäßrig-alkalischen Entwicklern nicht ausreichend löslich.

Ein alternatives chloridfreies Herstellungsverfahren für Poly-o-hydroxyamide besteht darin, zur Umsetzung der Dicarbonsäure mit dem Bis-o-aminophenol Kondensationsreagenzien wie 1-Ethoxycarbonyl-2-ethoxy-1,2-dihydrochinolin und 1,1'-Carbonyldioxy-di-1,2,3-benzotriazol einzusetzen (siehe: EP-OS 0 391 196). Die auf diese Weise hergestellten Polymere zeigen aber in metallionenfreien wäßrig-alkalischen Entwicklern ebenfalls nur eine unzureichende Löslichkeit.

Es sind auch Verfahren bekannt, bei denen die Amidbildung mittels Phosphorverbindungen erfolgt (siehe dazu: EP-OS 0 481 402, US-PS 4 331 592 und DE-OS 37 16 629). Bei Poly-o-hydroxyamiden führen derartige Synthesen aber entweder zu cyclisierten, d.h. alkaliunlöslichen Produkten oder es verbleiben im Polymer phosphorhaltige, teilweise auch chemisch gebundene Reste, wodurch das Polymer, wegen der Dotierungseigenschaften von Phosphor, für mikroelektronische Anwendungen unbrauchbar wird. Im Gegensatz zu ionischen Verunreinigungen können nämlich derartige Reste beispielsweise nicht mittels Ionenaustauscher entfernt werden.

Neben der ausreichenden Löslichkeit in metallionenfreien wäßrig-alkalischen Entwicklern müssen Polybenzoxazol- und Polybenzothiazol-Vorstufen auch eine gute Löslichkeit in verschiedenen nicht-toxischen organischen Lösungsmitteln aufweisen. Die Löslichkeit in organischen Lösungsmitteln kann beispielsweise durch Acetylierung von Hydroxylgruppen verbessert werden (siehe dazu: EP-OS 0 264 678), dadurch wird aber gleichzeitig die Löslichkeit in alkalischen Entwicklern verringert.

Aufgabe der Erfindung ist es, Dicarbonsäurederivate bereitzustellen, welche die Herstellung von Poly-o-hydroxyamiden und Poly-o-mercaptoamiden erlauben, die sowohl in metallionenfreien wäßrig-alkalischen Entwicklern als auch in nicht-toxischen organischen Lösungsmitteln gut löslich sind.

Dies wird erfindungsgemäß durch Dicarbonsäurederivate der Struktur 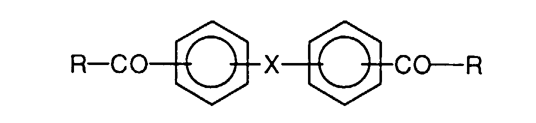 erreicht, wobei folgendes gilt:
- X =: O, S, (CF₂)ₘ, C(CF₃)₂ oder CF₂-CF(CF₃)
mit m = 1 bis 10;
- R: hat folgende Bedeutung: 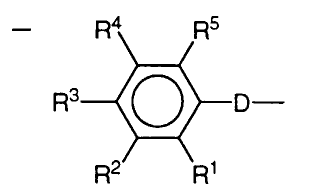
mit
- D =: O, S oder NH
und
- R¹, R², R³, R⁴, R⁵ =: H, F, CH₃ oder CF₃ (unabhängig voneinander), wobei mindestens einer der Reste R¹ bis R⁵ F bzw. CF₃ ist und höchstens zwei der Reste R¹ bis R⁵ CH₃ bzw. CF₃ sind; 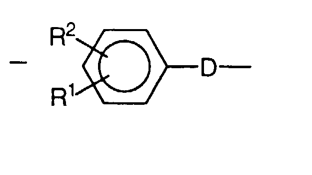
mit D = O oder S
und R¹, R² = H, F, CH₃, CF₃ oder CN (unabhängig voneinander), wobei mindestens einer der Reste R¹ und R² CN ist; 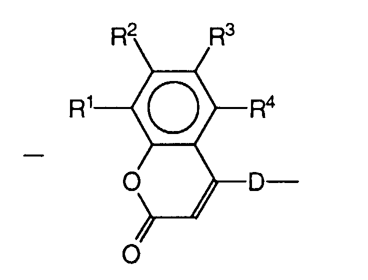 mit D = O, S oder NH
und R¹, R², R³, R⁴ = H, F, CH₃ oder CF₃ (unabhängig voneinander), wobei höchstens zwei der Reste R¹ bis R⁴ CH₃ bzw. CF₃ sind; 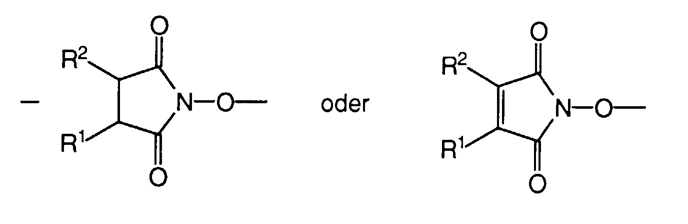 mit R¹, R² = H, F, CH₃ oder CF₃ (unabhängig voneinander); 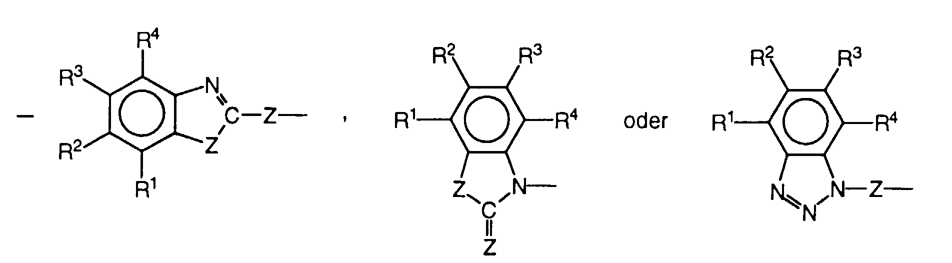 mit Z = O oder S
und R¹, R², R³, R⁴ = H, F, CH₃ oder CF₃ (unabhängig voneinander), wobei höchstens zwei der Reste R¹ bis R⁴ CH₃ bzw. CF₃ sind.

Die Dicarbonsäurederivate nach der Erfindung sind Diester, Dithioester und Diamide, d.h. sogenannte aktive Säurederivate. Diese aktiven Ester, Thioester und Amide leiten sich von folgenden Verbindungen ab: Fluor- bzw. Trifluormethyl- und Cyanophenole, -thiophenole oder -aminobenzole, 4-Hydroxy-, 4-Mercapto- und 4-Aminocumarine, N-Hydroxysuccin- und N-Hydroxymaleinimide, 2-Hydroxy- und 2-Mercaptobenzoxazole bzw. -benzothiazole sowie 1-Hydroxy- und 1-Mercaptobenzotriazole. Diese Komponenten bewirken, daß die Dicarbonsäurederivate nach der Erfindung bei der Umsetzung mit Bis-o-amino(thio)phenolen eine selektive Reaktivität entfalten, d.h. es erfolgt eine Amidbildung, aber keine Esterbildung.

Aktive Ester sind an sich bekannt, und zwar auf der Basis von Aminosäuren (siehe dazu: DE-OS 22 63 502 und "Chemische Berichte", Bd. 106 (1973), Seiten 3626 bis 3635) sowie von aliphatischen Dicarbonsäuren und Isophthalsäure (siehe dazu: "Polymer Science U.S.S.R.", Vol. 26 (1984), No. 7, Seiten 1668 bis 1678). Aktive Ester auf der Basis von Dicarbonsäuren, wie sie durch die Erfindung bereitgestellt werden, wurden bislang aber noch nicht beschrieben.

Die aktiven Dicarbonsäurederivate nach der Erfindung zeichnen sich dadurch aus, daß sie in der Säurekomponente zwei aromatische Kerne aufweisen, die durch eine spezielle Brücke miteinander verbunden sind, nämlich durch eine Gruppierung folgender Art: -O-, -S-, -(CF₂)ₘ-, -C(CF₃)₂- oder -CF₂-CF(CF₃)-.

Der Vorteil dieser Brücken, die flexibel sind, besteht darin, daß sie eine löslichkeitsverbessernde Wirkung entfalten. Außerdem kann damit auch die Haftung, die Lagerstabilität und die UV-Transparenz verbessert werden. Andere Brücken, wie -CH₂- und -CO-, können zwar ebenfalls die Löslichkeit verbessern, sie verschlechtern gleichzeitig aber andere Eigenschaften, wie thermische Beständigkeit oder UV-Transparenz.

Eine besonders geeignete Brücke ist die Gruppierung -O-, beispielsweise in der Diphenylether-4,4'-dicarbonsäure. Besonders geeignete aktive Ester nach der Erfindung sind deshalb: 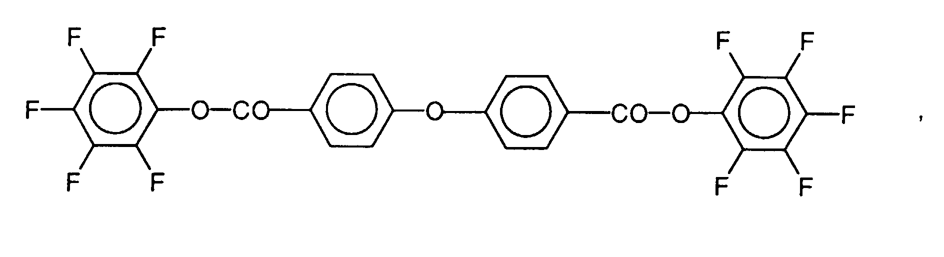 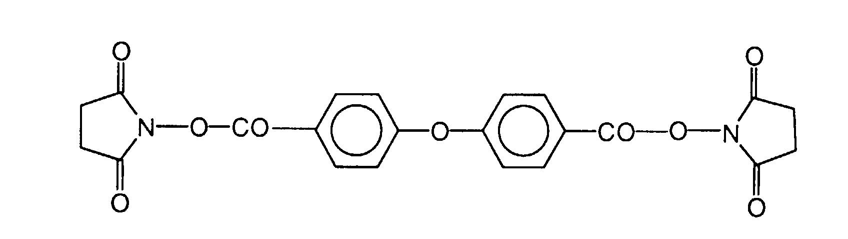 und

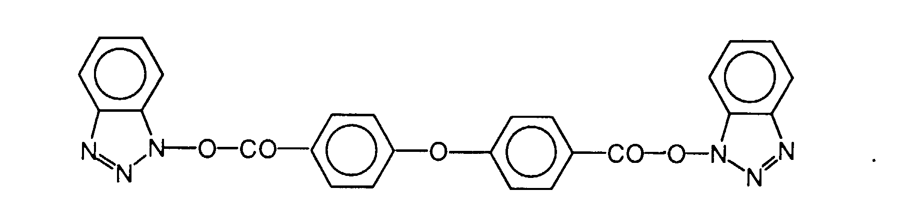

Die Dicarbonsäurederivate nach der Erfindung werden vorteilhaft in der Weise hergestellt, daß eine Dicarbonsäure der Struktur 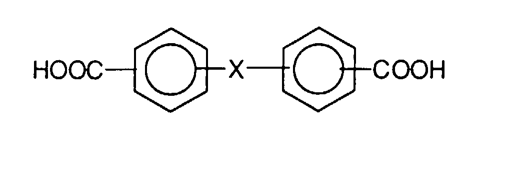 in Gegenwart eines Carbodiimids, vorzugsweise Dicyclohexylcarbodiimid, mit einer Verbindung der Struktur

**R-H**

zur Reaktion gebracht wird. Die Reste X und R haben dabei die vorstehend angegebene Bedeutung. Das H-Atom in der Verbindung RH ist an ein Sauerstoff-, Schwefel- oder Stickstoffatom gebunden und somit ein "aktiviertes" Wasserstoffatom.

Zur Herstellung der Dicarbonsäurederivate können die Verbindungen RH aber auch mit Dicarbonsäurechloriden umgesetzt werden. Die Reaktionsprodukte lassen sich dabei auf einfache Weise durch Umkristallisation in ausreichendem Maße reinigen.

Die aktiven Dicarbonsäurederivate nach der Erfindung ermöglichen die Herstellung von Polybenzoxazol- bzw. Polybenzothiazol-Vorstufen unter Ausschluß von Verunreinigungen, wie Chlorid- und Metallionen sowie Phosphorverbindungen, die auf elektronische Bauelemente eine schädigende Wirkung ausüben. Die Umsetzung mit den Bis-o-amino(thio)phenolen erfolgt dabei vorzugsweise in Gegenwart eines basischen Katalysators, insbesondere eines tertiären Amins, wie Pyridin und Triethylamin. Die auf diese Weise hergestellten Vorstufen sind in metallionenfreien wäßrig-alkalischen Entwicklern gut löslich, d.h. die Reaktionsselektivität der Amidbildung ist ausreichend hoch, und sie eignen sich somit als Basisharz für photosensitive Dielektrika. Ferner sind die Vorstufen in einer Reihe von organischen Lösungsmitteln gut löslich.

Anhand von Ausführungsbeispielen soll die Erfindung noch näher erläutert werden.

### Beispiel 1

### Herstellung eines Diesters aus Diphenylether-4,4'-dicarbonsäure und Pentafluorphenol

In einem 1 l-Dreihalskolben werden unter Stickstoff (als Schutzgas) 20,0 g Diphenylether-4,4'-dicarbonsäure (77,5 mmol) und 28,5 g Pentafluorphenol (155 mmol) in 270 ml trockenem N-Methylpyrrolidon unter Rühren gelöst. Die dabei erhaltene Lösung wird auf -15°C abgekühlt, dann wird eine Lösung von 32,0 g Dicyclohexylcarbodiimid (155 mmol) in 175 ml N-Methylpyrrolidon langsam zugetropft. Nach beendeter Zugabe wird die Reaktionslösung zunächst 2 h bei ca. -15°C, dann 14 h bei ca. 23°C und anschließend 5 h bei ca. 50°C gerührt. Nach dem Abkühlen auf Raumtemperatur wird die Reaktionslösung für ca. 18 bis 20 h stehengelassen. Danach wird das ausgefallene Produkt (Dicyclohexylharnstoff) abfiltriert und die klare Reaktionslösung unter starkem Rühren langsam in 1600 ml Wasser getropft. Der dabei abgeschiedene Feststoff wird abfiltriert, dreimal mit je 50 ml Wasser gewaschen und mit 500 ml Isopropanol versetzt. Anschließend wird ca. 20 h gerührt, filtriert und dann dreimal mit je 50 ml Isopropanol gewaschen. Nachfolgend wird zunächst 24 h bei ca. 20 mbar und dann 48 h bei ca. 2 mbar, jeweils bei 30°C, getrocknet. Der auf diese Weise hergestellte Diester (Ausbeute: 28 g) hat eine Schmelztemperatur von 175°C.

### Beispiel 2

### Herstellung eines Diesters aus Diphenylether-4,4'-dicarbonsäure und N-Hydroxysuccinimid

In einem 1 l-Dreihalskolben werden unter Stickstoff (als Schutzgas) 30,0 g Diphenylether-4,4'-dicarbonsäure (116,1 mmol) und 26,7 g N-Hydroxysuccinimid (232,2 mmol) in 310 ml trockenem N-Methylpyrrolidon unter Rühren gelöst. Die dabei erhaltene Lösung wird auf -15°C abgekühlt, dann wird eine Lösung von 47,9 g Dicyclohexylcarbodiimid (232,2 mmol) in 265 ml N-Methylpyrrolidon langsam zugetropft. Nach beendeter Zugabe wird die Reaktionslösung zunächst 2 h bei ca. -15°C, dann 24 h bei ca. 23°C und anschließend 6 h bei ca. 80°C gerührt. Nach dem Abkühlen auf Raumtemperatur wird die Reaktionslösung für ca. 18 bis 20 h stehengelassen. Danach wird das ausgefallene Produkt (Dicyclohexylharnstoff) abfiltriert und die klare Reaktionslösung unter starkem Rühren langsam in 3000 ml einer Mischung aus drei Teilen Wasser und einem Teil Essigsäure getropft. Der dabei abgeschiedene Feststoff wird abfiltriert und dreimal mit je 50 ml Wasser gewaschen. Der Feststoff wird dann zuerst 24 h in 500 ml Ethylacetat und danach 18 bis 20 h in 500 ml Isopropanol gerührt. Anschließend wird filtriert und dann dreimal mit je 50 ml Isopropanol gewaschen. Nachfolgend wird zunächst 24 h bei ca. 20 mbar und dann 48 h bei ca. 2 mbar, jeweils bei 30°C, getrocknet. Der auf diese Weise hergestellte Diester (Ausbeute: 34 g) hat eine Schmelztemperatur von 185°C.

### Beispiel 3

### Herstellung eines Diesters aus Diphenylether-4,4'-dicarbonsäure und 1-Hydroxybenzotriazol

In einem 4 l-Dreihalskolben werden unter Stickstoff (als Schutzgas) 204 g Diphenylether-4,4'-dicarbonsäure (0,79 mol) und 268 g 1-Hydroxybenzotriazol (1,984 mol) in 800 ml trockenem N-Methylpyrrolidon unter Rühren gelöst. Zu dieser Lösung wird unter Eiskühlung - bei einer Innentemperatur von ca. 0°C - eine Lösung von 456 g Dicyclohexylcarbodiimid (2,21 mol) in 800 ml N-Methylpyrrolidon innerhalb von 3 h zugetropft. Nach beendeter Zugabe wird die Reaktionslösung 2 h bei ca. 0°C gerührt, dann werden weitere 800 ml N-Methylpyrrolidon zugegeben, und die Lösung wird für 20 h bei ca. 23°C stehengelassen. Danach wird die Reaktionslösung wieder auf ca. 0°C abgekühlt, das ausgefallene Produkt (Dicyclohexylharnstoff) abfiltriert und viermal mit je 100 ml kaltem N-Methylpyrrolidon gewaschen. Das klare Filtrat wird mit N-Methylpyrrolidon auf insgesamt 4 l verdünnt und dann werden unter starkem Rühren 800 ml destilliertes Wasser langsam getropft. Der dabei abgeschiedene Feststoff wird abfiltriert, viermal mit je 200 ml Isopropanol gewaschen und mit 1500 ml Isopropanol aufgeschlämmt. Dann wird 15 min unter Rückfluß erhitzt, auf Raumtemperatur abgekühlt und filtriert. Nachfolgend wird zunächst 24 h bei ca. 20 mbar und dann 48 h bei ca. 2 mbar, jeweils bei 30°C, getrocknet. Der auf diese Weise hergestellte Diester (Ausbeute: 175 g) hat eine Schmelztemperatur von 198°C.

## Patentansprüche

1. Dicarbonsäurederivate der Struktur 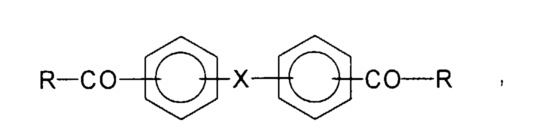 wobei folgendes gilt:
X = O, S, (CF₂)ₘ, C(CF₃)₂ oder CF₂-CF(CF₃)
mit m = 1 bis 10;
R hat folgende Bedeutung: 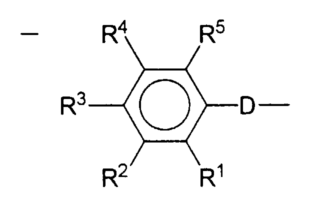 mit
D = O, S oder NH
und
R¹, R², R³, R⁴, R⁵ = H, F, CH₃ oder CF₃ (unabhängig voneinander), wobei mindestens einer der Reste R¹ bis R⁵ F bzw. CF₃ ist und höchstens zwei der Reste R¹ bis R⁵ CH₃ bzw. CF₃ sind; 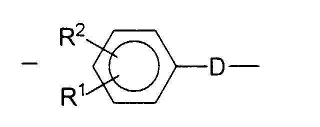
mit
D = O oder S
und
R¹, R² = H, F, CH₃, CF₃, oder CN (unabhängig voneinander), wobei mindestens einer der Reste R¹ und R² CN ist; 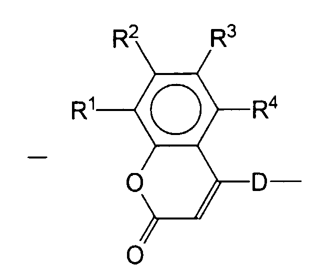
mit
D = O, S oder NH
und
R¹, R², R³, R⁴ = H, F, CH₃ oder CF₃ (unabhängig voneinander), wobei höchstens zwei der Reste R¹ bis R⁴ CH₃ bzw. CF₃ sind; 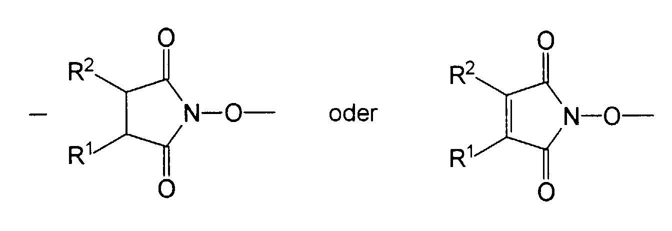
mit R¹, R² = H, F, CH₃ oder CF₃ (unabhängig voneinander); 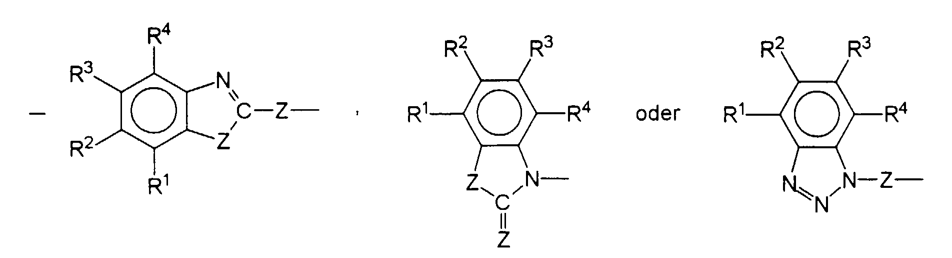
mit
Z = O oder S
und
R¹, R², R³, R⁴ = H, F, CH₃ oder CF₃ (unabhängig voneinander), wobei höchstens zwei der Reste R¹ bis R⁴ CH₃ bzw. CF₃ sind.

2. Dicarbonsäureester der Struktur 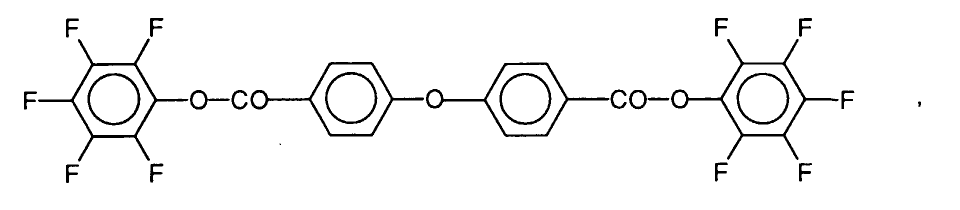 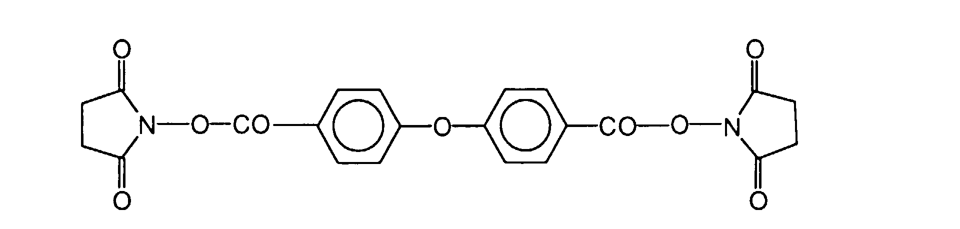 oder

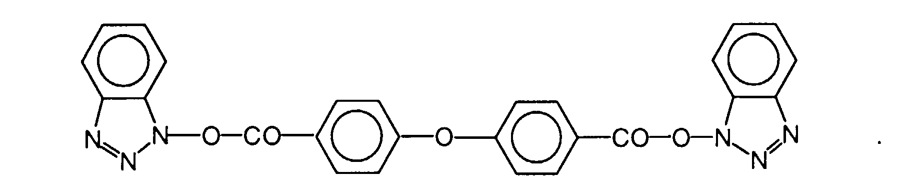

3. Verfahren zur Herstellung von Dicarbonsäurederivaten nach Anspruch 1 oder 2, **dadurch gekennzeichnet**, daß eine Dicarbonsäure der Struktur 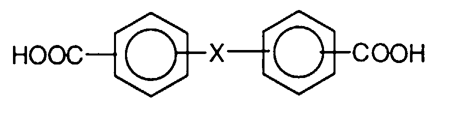 in Gegenwart eines Carbodiimids mit einer Verbindung der Struktur
**R-H**
zur Reaktion gebracht wird, wobei X und R die im Anspruch 1 angegebene Bedeutung haben.

4. Verfahren nach Anspruch 3, **dadurch gekennzeichnet,** daß die Reaktion in Gegenwart von Dicyclohexylcarbodiimid durchgeführt wird.

5. Verwendung der Dicarbonsäurederivate nach Anspruch 1 oder 2 zur Herstellung von Poly-o-hydroxyamiden und Poly-o-mercaptoamiden durch Umsetzung mit einem Bis-o-aminophenol bzw. Bis-o-aminothiophenol.

## Claims

1. Dicarboxylic acid derivatives of the structure 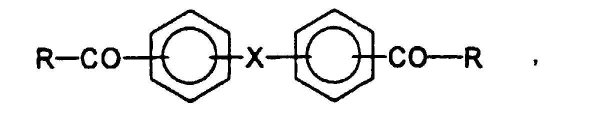 where:
X = O, S, (CF₂)ₘ, C(CF₃)₂ or CF₂-CF(CF₃)
where m = 1 to 10;
R is: 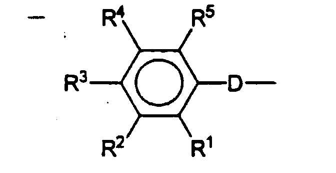 where
D = O, S or NH
and
R¹, R², R³, R⁴, R⁵ = H, F, CH₃ or CF₃ (independently of one another), where at least one of the radicals R¹ to R⁵ is F or CF₃ and not more than two of the radicals R¹ to R⁵ are CH₃ or CF₃; 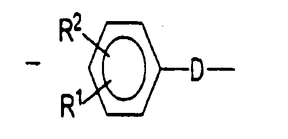
where
D = O or S
and
R¹, R² = H, F, CH₃, CF₃ or CN (independently of one another), where at least one of the radicals R¹ and R² is CN; 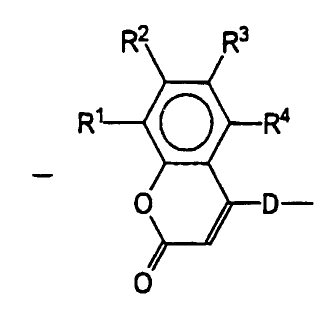
where D = O, S or NH
and
R¹, R², R³, R⁴ = H, F, CH₃ or CF₃ (independently of one another), where not more than two of the radicals R¹ to R⁴ are CH₃ or CF₃; 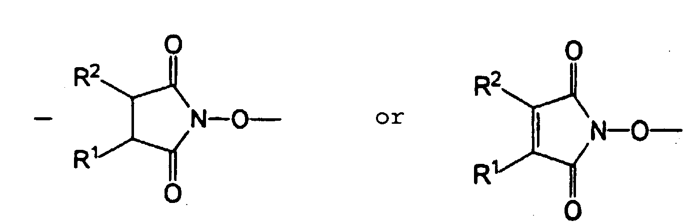
where R¹, R² = H, F, CH₃ or CF₃ (independently of one another); 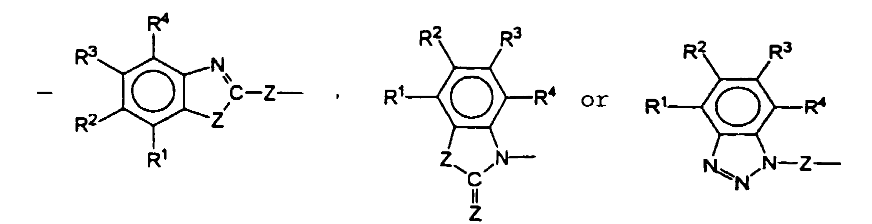
where Z = O or S
and
R¹, R², R³, R⁴ = H, F, CH₃ or CF₃ (independently of one another), where not more than two of the radicals R¹ to R⁴ are CH₃ or CF₃.

2. Dicarboxylic esters of the structure 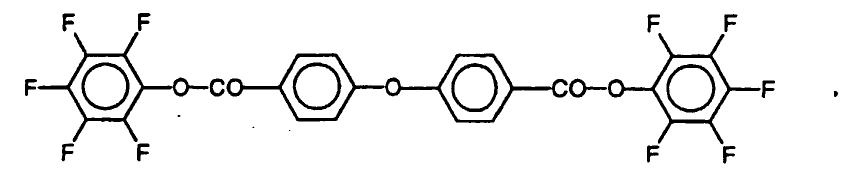 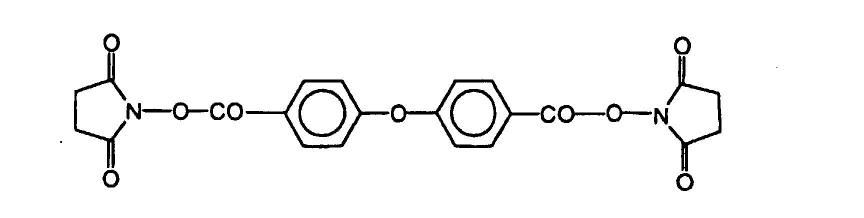 or

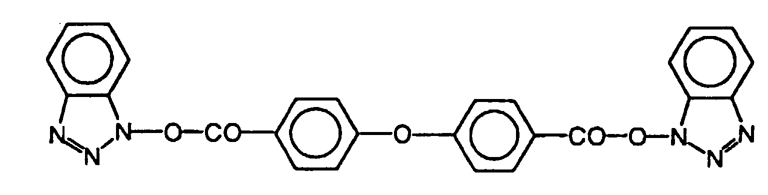

3. Process for preparing dicarboxylic acid derivatives according to Claim 1 or 2, characterized in that a dicarboxylic acid of the structure 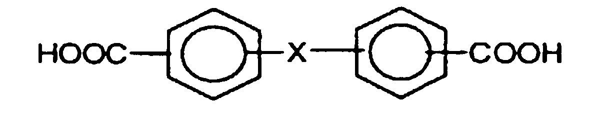 is reacted in the presence of a carbodiimide with a compound of the structure
**R-H,**
where X and R are as defined in Claim 1.

4. Process according to Claim 3, characterized in that the reaction is carried out in the presence of dicyclohexylcarbodiimide.

5. Use of the dicarboxylic acid derivatives according to Claim 1 or 2 for preparing poly-o-hydroxyamides and poly-o-mercaptoamides by reaction with a bis-o-aminophenol or bis-o-aminothiophenol.

## Revendications

1. Dérivés d'acide dicarboxylique présentant la structure 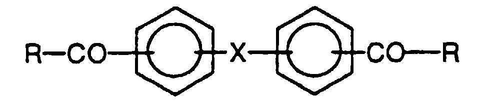 avec les significations suivantes :
X = O, S, (CF₂)ₘ, C(CF₃)₂ ou CF₂-CF(CF₃) avec m = 1 à 10 ;
R présente la signification suivante : 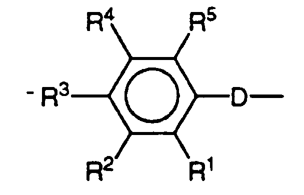
avec D = O, S ou NH
et
R¹, R², R³, R⁴, R⁵ = H, F, CH₃ ou CF₃ (indépendamment les uns des autres), où au moins un des résidus R¹ à R⁵ représente F, respectivement CF₃ et au maximum deux des résidus R¹ à R⁵ représentent CH₃, respectivement CF₃ ; 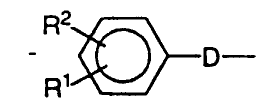
avec D = O ou S
et
R¹, R² = H, F, CH₃, CF₃ ou CN (indépendamment l'un de l'autre), où au moins un des résidus R¹ et R² représente CN ; 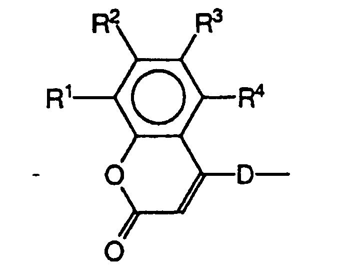
avec D = O, S ou NH
et
R¹, R², R³, R⁴ = H, F, CH₃ ou CF₃ (indépendamment les uns des autres), où au maximum deux des résidus R¹ à R⁴ représentent CH₃, respectivement CF₃; 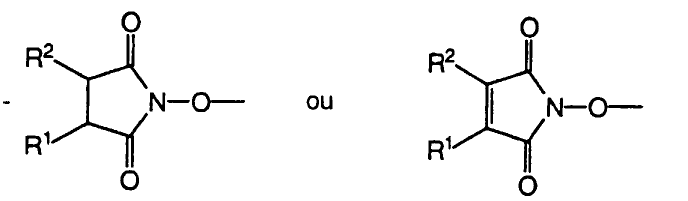
avec R¹, R² = H, F, CH₃ ou CF₃ (indépendamment l'un de l'autre) ; 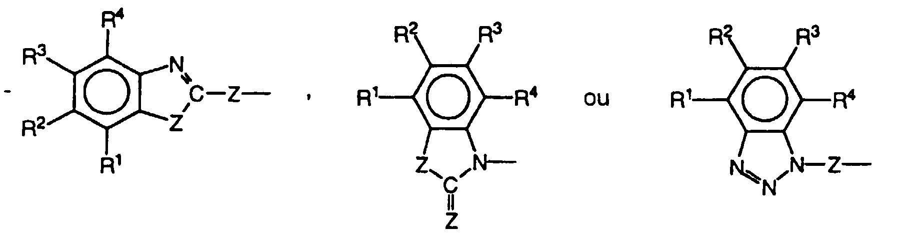
avec Z = O ou S
et
R¹, R², R³, R⁴ = H, F, CH₃ ou CF₃ (indépendamment les uns des autres), où au maximum deux des résidus R¹ à R⁴ représentent CH₃, respectivement CF₃.

2. Esters d'acide dicarboxylique présentant la structure 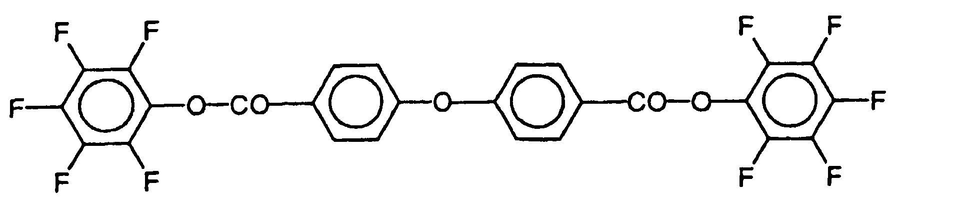 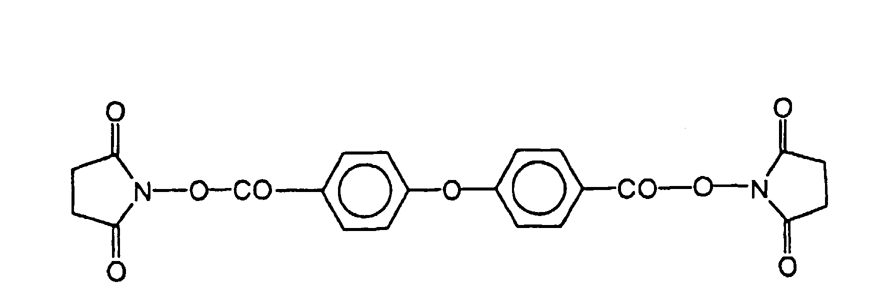 ou

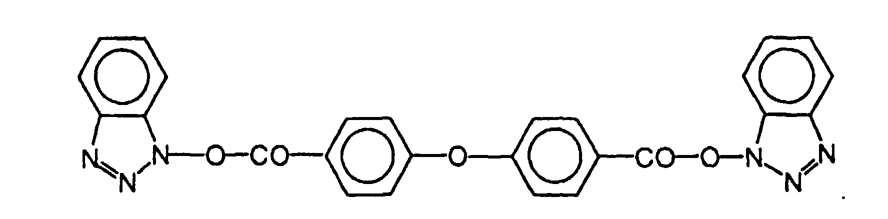

3. Procédé de préparation de dérivés d'acide dicarboxylique selon la revendication 1 ou 2, caractérisé en ce qu'un acide dicarboxylique présentant la structure 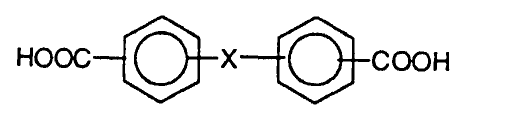 est mis à réagir en présence d'un carbodiimide avec un composé présentant la structure
R-H,
X et R présentant la signification indiquée à la revendication 1.

4. Procédé selon la revendication 3, caractérisé en ce que la réaction est effectuée en présence de dicyclohexylcarbodiimide.

5. Utilisation de dérivés d'acide dicarboxylique selon la revendication 1 ou 2 pour la préparation de poly-o-hydroxyamides et de poly-o-mercaptoamides par réaction avec un bis-o-aminophénol, respectivement bis-o-aminothiophénol.
